# EUROPEAN PATENT APPLICATION

(11) **EP 2 759 293 A1**
(43) Date of publication of application: **30.07.2014**
(21) Application number: 13152687.3
(22) Date of filing: 25.01.2013
(51) Int. Cl.: A61K 9/16, A61K 9/20, A61K 31/192, A61P 29/00

(54) **Pharmaceutical compositions of (+)-(s)-2-(3-benzoylphenyl)propionic acid tromethamine salt**

(71) Applicant: Laboratorios Lesvi, S.L., 08970 Sant Joan Despi (ES)
(72) Inventor: Díez Martín, Ignacio, 08970 San Joan Despi (ES); Úbeda Pérez, Carmen, 08970 San Joan Despi (ES); Costa Subira, Josep, 08970 San Joan Despi (ES)
(74) Representative: Isarpatent

(57) **Abstract**

This invention relates to solid oral pharmaceutical compositions of (+)-(S)-2-(3-Benzoylphenyl)propionic acid tromethamine salt; a process of the manufacturing thereof and their use in the treatment of pain and inflammation.

## Description

### FIELD OF THE INVENTION

This invention relates to solid oral pharmaceutical compositions of (+)-(*S*)-2-(3-Benzoylphenyl)propionic acid tromethamine salt.

### BACKGROUND OF THE INVENTION

(+)-(*S*)-2-(3-Benzoylphenyl)propionic acid, also known as Dexketoprofen, is the S-enantiomer of ketoprofen, a non-steroidal anti-inflammatory drug (NSAID). Dexketoprofen is also a non-steroidal anti-inflammatory drug (NSAID) having a potent analgesic and antipyretic action, and eliciting a faster and more potent analgesic response than ketoprofen in its racemic form.

The tromethamine salt of Dexketoprofen, which is also known as dexketoprofen trometamol, was first disclosed in WO0941332 A1, and has the following structure:

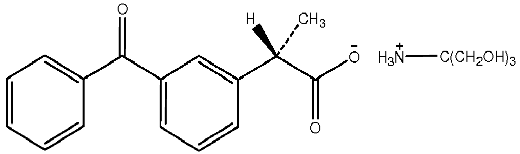

According to WO09411332 A1, the dexketoprofen trometamol presents high and fast solubility in water (>100% w/v), which is also higher and faster than any other dexketoprofen salt. Said characteristics allow the compound to be administered intramuscularly or intravenously, or as highly soluble tablets having a very fast dissolution rate. In addition to the ease of administration, the dexketoprofen trometamol exhibits a faster onset of analgesic action, an enhanced analgesic response and a longer duration than racemic ketoprofen or other salts of dexketoprofen.

EP 1739072 A1 discloses two polymorphs of dexketoprofen trometamol, named form A and form B. According to EP 1739072 A1, the polymorphic form B is less stable than the form A and converts gradually and spontaneously into the form A over a period of several weeks or months. Formulations of oral solid forms comprising dexketoprofen trometamol form A, particularly tablets, are disclosed and obtained by direct compression methods, avoiding a wet granulation step which may affect the stability of the polymorphic form. In addition, Blanco et al., Analytica Chimica Acta, 567 (2006) 262-268 describes tablets obtained by granulation in an aqueous medium, in which both dexketoprofen trometamol polymorphs form A or form B, evolved to the amorphous form, irrespectively of the initial crystalline form present.

The present inventors have tried to reproduce the preparation of the tablets of dexketoprofen trometamol form A containing standard excipients such as microcrystalline cellulose and lactose by direct compression, as described in EP 1739072 A1 and they have surprisingly found that the compositions described in EP 1739072 A1 are unsuitable for tableting by direct compression, since the flowability of said compositions is severely impaired.

In accordance with regulatory requirements of the U.S. and other countries, e.g. the FDA's Good Manufacturing Practice ("GMP") requirements, when preparing pharmaceutical compositions containing active pharmaceutical ingredients for administration to mammals, there is a need to produce crystalline forms, or polymorphs, which are as pure and as stable as possible. Differences in the chemical and physical properties of polymorphic forms of active pharmaceutical ingredients can have a direct effect on the ability to consistently manufacture its pharmaceutical compositions, as well as on its stability, dissolution rate and the bioavailability of said pharmaceutical compositions. As a result, a manufacturing process that provides pharmaceutical compositions containing unstable polymorphic forms is not desirable.

Therefore, it is highly desirable to develop pharmaceutical compositions of dexketoprofen trometamol characterised by having high chemical and polymorphic stability over time, good flowability and improved dissolution rate, regardless of the particle size distribution of the dexketoprofen trometamol used.

### DEFINITIONS

The term "dosage form" as used herein refers to a mixture of the active pharmaceutical ingredient and the non-active components or excipients. There are several types of dosage forms, including liquid dosage forms, solid dosage forms and semisolid dosage forms, which can be also be differentiate by the method of administration. The dosage forms of the pharmaceutical composition of the present invention corresponds to oral solid dosage forms. Preferably said oral dosage forms are tablets.

The term "granulate" as used herein refers to an active pharmaceutical ingredient and/or excipients present in the formulation forming granules; defining granules as small particles gathered into larger, permanent aggregates in which the original particles can still be identified.

The term "extragranular phase" as used herein refers to the excipients present in the formulation which are out of the granulate.

The term "dry granulation" as used herein refers to a process to form granules without using a liquid solution. Dry granulation can be carried out by slugging or by roller compaction processes. Slugging is a process, wherein the material to be granulated is compressed to a large compressed mass, compact, or "slug". Roller compaction is a process wherein the material to be granulated passes between high-pressure rollers that compress it, forming a compact or sheet. The resulting material of the slugging or the roller compaction processes is reduced to obtain an uniform size granulate by means of standard industrial techniques such as milling or other equivalent processes. The obtained granulate can be blended with the extragranular phase and compressed into tablets by a second compression process. Dry granulation process results in a product that contains minimal amounts of water. The amount of water in the final formulations, as opposed to the granulates, is a function of granulate water as well as minor amounts of water used during subsequent process steps such as coating. These amounts of water added in later steps than granulation generally will not affect the stability of the active pharmaceutical ingredients, and therefore are subject to considerable permitted variation. Dry granulation can also be referred to as precompression or double-compression.

The term "coating" as used herein refers to adherence, adsorption, loading and/or incorporation, to some extent, preferable uniformly, of at least one solution, dispersion or suspension coating material onto and/or into a substrate. Preferably the coating material is an aqueous dispersion or suspension. The coating material on the substrate may be of any thickness. Preferably the coating material is a thin and uniform film applied onto the substrate.

The term "film coating" as used herein refers to a thin and uniform film applied onto a substrate. The film coating according to the present invention comprises at least a film forming agent, which can be any polymer appropriate for film coating. Examples of appropriate polymers/film forming agents include, but are not limited to cellulose derivatives such as methylcellulose (MC), carboxymethylcellulose (CMC), sodium carboxymethylcellulose, hydroxymethylcellulose (HMC), hydroxypropylcellulose (HPC), hydroxyethyl cellulose (HEC), or hydroxypropyl methylcellulose (HPMC, hypromellose); acrylics such as methacrylate; or methyl methacrylate copolymers; vinyls such as polyvinyl alcohol; and mixtures thereof. The film coating according to the present invention may further comprise a filler and a plasticiser. The filler is preferably selected from lactose, fructose, polydextrose, sucrose, maltose and mixtures thereof. The plasticiser is preferably selected from citrate esters (e.g. triethylcitrate, triacetin); low molecular weight polyalkylene oxides (e.g. polyethylene glycols, polypropylene glycols, polyethylene/propylene glycols); glycerol, pentaerythritol, glycerol monoacetate, diacetate or triacetate, propylene glycol, and sodium diethyl sulfosuccinate. The procedures that can be used for film coating are spraying or spray-drying by means of coating pan, spray or fluidized coating equipment. Preferably the film coating is performed by spraying using a coating pan.

The term "particle size distribution" as used herein refers to the relative percentages by volume of each of the different size fractions of a particulate matter. The particle size distributions of the present application can be measured using laser light diffraction equipment, such as Malvern Mastersizer® 2000. Particle size is determined by measuring the angular distribution of laser light scattered by a homogeneous suspension of particles. The size distribution is determined from the light scattering data using the theory of light scattering developed by Gustav Mie. Other types of equipment are also suitable to determine particle size distribution. Laser light diffraction results can be expressed by d50, and/or d90 values, which are based on a volume distribution. The term "dx", as used herein, means that x% of the particles in a composition (based on volume) have a diameter equal to or below a specified d value. Thus, a d90 of 300 µm means that 90% by volume, of the particles, have a diameter equal to or below 300 µm. In addition to using d90 as a measuring reference to determine particle size, d50 is also used for such purpose. Likewise, a d50 of 30 µm means that 50%, by volume, of the particles, have a diameter equal to or below 30 µm.

The term "flowability" as used herein refers to the ability of divided solids (for example powders and granules) to flow vertically under defined conditions. As stated in European Pharmacopeia 7.0.(2.9.16., monograph) the flowability is determined by measuring the time needed for a test sample to flow out of a funnel, which can have different angle and/or orifice diameter according to the flow properties of the material to be tested. Typical orifice diameters of the funnel are 10 mm, 15 mm and 25 mm. The flowability is expressed in seconds and tenths of seconds, related to 100 g of sample.

The term "dosage uniformity" as used herein refers to the degree of uniformity in the amount of the active substance among dosage units; defining dosage units as dosage forms containing a single dose or a part of a dose of the active substance in each dosage unit. To ensure the dosage uniformity, each dosage unit should have an active substance content within a narrow range around the label claim. The dosage uniformity of the pharmaceutical compositions according to the present invention can be demonstrated by means of performing the test for content uniformity, as states in European Pharmacopeia (2.9.40, monograph). As defined therein, the requirements for dosage uniformity are met if the acceptance value of the dosage units is less than or equal to 15. The acceptance value (AV) is a figure which characterized the dosage uniformity. The acceptance value is calculated, as stated in the European Pharmacopeia 7.0. (2.9.40, monograph).

The term "lubricant" as used herein is defined as an agent able to decrease adhesion of a powder to punches and friction between particles. Non-limiting examples of lubricants include magnesium stearate, calcium stearate, zinc stearate, sucrose stearate, glyceryl palmitostearate, sodium benzoate, talc, and mixtures thereof. Preferably, the lubricant is selected from magnesium stearate, sucrose stearate, glyceryl palmitostearate, and mixtures thereof. More preferably the lubricant is magnesium stearate.

The term "diluent" as used herein is defined as an agent able to increase the bulk of a solid pharmaceutical composition, and may make a pharmaceutical dosage form containing the composition easier for the patient and/or a care giver to handle. Non-limiting examples of diluents are saccharides (monosaccharides, oligosaccharides or polysaccharides), and/or their oxidised and/or reduced forms, such as sucrose, fructose, polydextrose, sucrose, maltose, isomaltose and lactose in its various forms (anhydrous, monohydrate, agglomerated forms or atomised forms); sugar alcohols such as mannitol, , sorbitol, maltitol, xylitol, isomalt and erythritol; cellulose powder, microcrystalline cellulose, silicified microcrystalline cellulose and/or derivatives of cellulose modified chemically, such as hydroxypropyl cellulose, hydroxypropyl methyl cellulose; natural starches, such as maize starch and potato starch; pregelatinized starch; and mixtures thereof. Preferably, the diluent is selected from microcrystalline cellulose, silicified microcrystalline cellulose, maize starch, pregelatinized starch and mixtures thereof. More preferably, the diluent is selected from microcrystalline cellulose, pregelatinized starch and mixtures thereof. An equivalent term to "diluent" as used herein is also the term "filler".

The term "binder" as used herein is defined as an agent able to bind particles which cannot be bound only by a compression force. Non-limiting examples of binders include methylcellulose, hydroxypropyl cellulose, hydroxypropyl cellulose low substituted, hydroxyprpopyl methyl cellulose (hypromellose), macrogols, saccharose, starch, pregelatinized starch, and polyvinylpyrrolidone (povidone), and mixtures thereof. Preferably, the binder is selected from starch, pregelatinized starch, povidone and mixtures thereof. More preferably the binder is pregelatinized starch.

The term "disintegrant" as used herein refers to a compound which facilitates the break-up of a tablet when it is placed in aqueous environment. Disintegrants once in contact with water, swell, hydrate, change in volume or form to produce a disruptive force that opposes the efficiency of the binder/s causing the compressed table to break apart. Non-limiting examples of disintegrants include natural starches, such as maize starch and potato starch; modified starches, such as pregelatinized starch and sodium starch glycolate; natural or chemically-modified cellulose, especially crosslinked carboxymethyl cellulose (croscarmellose) or salts thereof, low substituted hydroxypropyl cellulose, hydroxyethyl cellulose; guar gum; alginic acid or salts thereof; synthetic polymers such as cross-linked polyvinylpyrrolidones, specially crospovidone; soy polysaccharides; derivatives of casein; and mixtures thereof. Preferably, the disintegrant is selected from pregelatinized starch, sodium starch glycolate, crosslinked carboxymethyl cellulose (croscarmellose) or salts thereof, cross-linked polyvinylpyrrolidones, specially crospovidone, and mixtures thereof. More preferably, the disintegrant is selected from pregelatinized starch, sodium starch glycolate, and mixtures thereof.

### BRIEF DESCRIPTION OF THE INVENTION

A first aspect of the present invention relates to a solid oral pharmaceutical composition of dexketoprofen trometamol, comprising:
(i) a granulate comprising dexketoprofen trometamol and at least one lubricant; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process.

A second aspect of the present invention is to provide a process for preparing the solid oral pharmaceutical composition of dexketoprofen trometamol of the invention.

Another aspect of the present invention relates to the use of the solid oral pharmaceutical composition of dexketoprofen trometamol of the invention for the treatment of pain and inflammation.

### DETAILED DESCRIPTION OF THE INVENTION

A first aspect of the present invention relates to a solid oral pharmaceutical composition of dexketoprofen trometamol, which affords good dosage uniformity, a good dissolution rate and good polymorphic and chemical stability. Said pharmaceutical composition comprising:
(i) a granulate comprising dexketoprofen trometamol and at least one lubricant; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process.

In a preferred embodiment of the present invention the solid oral pharmaceutical composition of the present invention is in the form of a tablet.

The granulate (i) in accordance with the first aspect of the present invention comprises dexketoprofen trometamol in an amount from 5% to 30%, preferably from 10% to 20% by weight relative to the total weight of the solid pharmaceutical composition. The granulate (i) also comprises at least one lubricant in an amount from 0.1% to 2.5%, preferably from 0.8% to 2% by weight relative to the total weight of the solid pharmaceutical composition. The lubricant may be selected from magnesium stearate, calcium stearate, zinc stearate, sucrose stearate, glyceryl palmitostearate, sodium benzoate, talc, and mixtures thereof. Preferably, the lubricant contained in the granulate (i) is selected from magnesium stearate, sucrose stearate, glyceryl palmitostearate and mixtures thereof. More preferably, the lubricant contained in the granulate (i) is magnesium stearate. Additionally, the granulate (i) may comprise one or more of the following pharmaceutical acceptable excipients: at least one diluent and/or at least one binder and/or at least one disintegrant. The diluent contained in the granulate (i) may be selected from saccharides and/or their oxidised and/or reduced forms, sugar alcohols, cellulose powder and/or derivatives of cellulose modified chemically, natural starches and/or pregelatinized starch, and mixtures thereof. The total amount of the diluent in the granulate (i) is between 0% and 80%, preferably between 20% and 80%, more preferably between 50% and 70%. Suitable diluents are lactose, mannitol, sorbitol, cellulose powder, microcrystalline cellulose, silicified microcrystalline cellulose, maize starch, pregelatinized starch and mixtures thereof. In a preferred embodiment, the diluent contained in the granulate (i) is present in an amount between 20% and 80%, and is selected from microcrystalline cellulose, silicified microcrystalline cellulose, maize starch, pregelatinized starch and mixtures thereof. In a more preferred embodiment, the diluent contained in the granulate (i) is microcrystalline cellulose, pregelatinized starch or mixtures thereof and is present in an amount between 50% and 70%. The binder contained in the granulate (i) may be selected from methylcellulose, hydroxypropyl cellulose, hydroxypropyl cellulose low substituted, hydroxyprpopyl methyl cellulose (hypromellose), macrogols, saccharose, starch, pregelatinized starch, polyvinylpyrrolidone (povidone), and mixtures thereof. The binder may be present in the granulate (i) in an amount between 0% and 15%, preferably between 5% and 15%, more preferably between 7% and 13%. In a preferred embodiment, the binder contained in the granulate (i) is present in an amount between 5% and 15% and is selected from methylcellulose, hydroxypropyl cellulose, hydroxypropyl cellulose low substituted, hydroxyprpopyl methyl cellulose (hypromellose), macrogols, saccharose, starch, pregelatinized starch, polyvinylpyrrolidone (povidone), and mixtures thereof. In a more preferred embodiment, the binder is present in an amount between 7% and 13%, and is starch, pregelatinized starch or mixtures thereof. The disintegrant contained in the granulate (i) may be selected from starch, pregelatinized starch, sodium starch glycolate, croscarmellose or salts thereof, crospovidone, and mixtures thereof. The disintegrant may be present in the granulate (i) in an amount between 0% and 20%, preferably between 5% and 20%, more preferably between 8% and 15%. In a preferred embodiment, the disintegrant contained in the granulate (i) is present in an amount between 8% and 15% and is selected from pregelatinized starch, sodium starch glycolate and mixtures thereof. Percentages are expressed by weight relative to the total weight of the solid pharmaceutical composition. The sum of all components adding to one hundred.

The dexketoprofen trometamol contained in the solid pharmaceutical composition of the present invention may be present, partially or completely, in different crystalline forms. In a particular embodiment, the dexketoprofen trometamol contained in the solid pharmaceutical composition of the present invention is present as particular polymorphic forms, such as the polymorphic form A and/or the polymorphic form B previously disclosed in the patent application EP1739072A1.

Surprisingly, it has been observed that the solid pharmaceutical composition of the present invention can comprise a dexketoprofen trometamol, which may have any particle size distribution. Suitable particle size distribution of the dexketoprofen trometamol, contained in the solid pharmaceutical composition of the present invention, is characterised by a d₉₀ equal or below 300 µm, preferably equal or below 150 µm, more preferably equal or below 75 µm.

The extragranular phase (ii) in accordance with the first aspect of the present invention comprises at least one diluent, at least one disintegrant and at least one lubricant. The diluent contained in the extragranular phase (ii) may be selected from saccharides and/or their oxidised and/or reduced forms, sugar alcohols, cellulose powder and/or derivatives of cellulose modified chemically, natural starches and/or pregelatinized starch, and mixtures thereof. The total amount of the diluent in the extragranular phase (ii) is between 4% and 80% (w/w), preferably between 5% and 30% (w/w), more preferably between 5% and 25% (w/w). In a preferred embodiment, the diluent contained in the extragranular phase (ii) is present in an amount between 4% and 80% (w/w), and is selected from lactose, mannitol, sorbitol, cellulose powder, microcrystalline cellulose, silicified microcrystalline cellulose, maize starch, pregelatinized starch and mixtures thereof. In a more preferred embodiment, the diluent contained in the extragranular phase (ii) is present in an amount between 5% and 30% (w/w), and is selected from microcrystalline cellulose, silicified microcrystalline cellulose, maize starch, pregelatinized starch and mixtures thereof. In the most preferred embodiment, the diluent contained in the extragranular phase (ii) is microcrystalline cellulose, pregelatinized starch or mixtures thereof, in an amount between 5% and 25 % (w/w). The disintegrant contained in the extragranular phase (ii) may be selected from starch, pregelatinized starch, sodium starch glycolate, croscarmellose or salts thereof, crospovidone, and mixtures thereof. The total amount of the disintegrant in the extragranular phase (ii) is between 0.5% and 10% (w/w), preferably between 1% and 5% (w/w). In a preferred embodiment, the disintegrant contained in the extragranular phase (ii) is present in an amount between 1% and 5% (w/w) and is selected from pregelatinized starch, sodium starch glycolate and mixtures thereof. The lubricant contained in the extragranular phase (ii) may be selected from magnesium stearate, calcium stearate, zinc stearate, sucrose stearate, glyceryl palmitostearate, sodium benzoate, talc, and mixtures thereof. The total amount of the lubricant in the extragranular phase (ii) is between 0.1% and 2.5% (w/w), preferably between 0.2% and 2% (w/w), more preferably between 0.5% and 1.7% (w/w). In a preferred embodiment, the lubricant contained in the extragranular phase (ii) is present in an amount between 0.2% and 2% (w/w) and is selected from magnesium stearate, sucrose stearate, glyceryl palmitostearate and mixtures thereof. In a more preferred embodiment, the lubricant contained in the extragranular phase (ii) is magnesium stearate in an amount between 0.5% and 1.7% (w/w). Additionally, the extragranular phase (ii) may comprise other pharmaceutical acceptable excipients such as binders. Accordingly, the extragranular phase (ii) may comprise at least one binder, which may be selected from methylcellulose, hydroxypropyl cellulose, hydroxypropyl cellulose low substituted, hydroxyprpopyl methyl cellulose (hypromellose), macrogols, saccharose, starch, pregelatinized starch, polyvinylpyrrolidone (povidone), and mixtures thereof. The binder may be present in the extragranular phase (ii) in an amount between 0% and 15% (w/w), preferably between 5% and 15% (w/w), more preferably between 7% and 13% (w/w). In a preferred embodiment, the binder contained in the extragranular phase (ii) is present in an amount between 5% and 15% (w/w)and is selected from methylcellulose, hydroxypropyl cellulose, hydroxypropyl cellulose low substituted, hydroxyprpopyl methyl cellulose (hypromellose), macrogols, saccharose, starch, pregelatinized starch, polyvinylpyrrolidone (povidone), and mixtures thereof. In a more preferred embodiment, the binder is present in an amount between 7% and 13% (w/w), and is starch, pregelatinized starch or mixtures thereof. Percentages are expressed by weight relative to the total weight of the solid pharmaceutical composition. The sum of all components adding to one hundred.

In a particular embodiment, the extragranular phase (ii) of the pharmaceutical composition according to the first aspect of the present invention comprises at least one diluent in an amount between 70% and 98% (w/w), preferably between 75% and 95% (w/w); at least one disintegrant in an amount between 3% and 20% (w/w), preferably between 5% and 20% (w/w); and at least one lubricant in an amount between 0.5% and 5% (w/w), preferably between 0.5% and 4.5% (w/w). Weight percentage by weight relative to the weight of the extragranular phase (ii). The sum of all components adding to one hundred.

In a preferred embodiment the solid pharmaceutical composition according to the first aspect of the present invention comprises the same lubricant in the granulate (i) as in the extragranular phase (ii).

In a particular embodiment, a film coating (iii) in accordance with the first aspect of the present invention may be used in order to improve the appearance of the solid pharmaceutical composition and to facilitate handling during the packaging of said solid pharmaceutical composition. The film coating (iii) of the solid oral pharmaceutical composition according to this embodiment can be prepared in-house or being a commercially available pre-formulated coating material, such as Opadry products. Said film coating comprises a film forming agent, which can be any polymer appropriate for film coating. Examples of appropriate polymers/film forming agents include, but are not limited to cellulose derivatives such as methylcellulose (MC), carboxymethylcellulose (CMC), sodium carboxymethylcellulose, hydroxymethylcellulose (HMC), hydroxypropylcellulose (HPC), hydroxyethyl cellulose (HEC), or hydroxypropyl methylcellulose (HPMC, hypromellose); acrylics such as methacrylate; or methyl methacrylate copolymers; vinyls such a as polyvinyl alcohol; and mixtures thereof. Preferably, the film forming agent, contained in the film coating, is a cellulose derivative or a vinyl; most preferible, the film forming agent is a cellulose deirvative selected from methyl cellulose, hydroxymethyl cellulose, hydroxyethyl cellulose, hydroxypropyl cellulose, hydroxypropyl methylcellulose, sodium carboxymethyl cellulose, and mixtures thereof. More preferably, the film forming agent, contained in the film coating (iii), is hydroxypropyl methylcellulose. The film coating (iii) of the pharmaceutical composition according to the first aspect of the present invention may further comprises a filler and a plasticiser. The filler is preferably selected from lactose, fructose, polydextrose, sucrose, maltose and mixtures thereof; more preferably the filler is polydextrose, lactose or mixtures thereof; most preferably, the filler is polydextrose. The plasticiser is preferably selected from citrate esters (e.g. triethylcitrate, triacetin); low molecular weight polyalkylene oxides (e.g. polyethylene glycols, polypropylene glycols, polyethylene/propylene glycols); glycerol, pentaerythritol, glycerol monoacetate, diacetate or triacetate, propylene glycol, and sodium diethyl sulfosuccinate. More preferably, the plasticiser is polyethylene glycol. The film coating may further comprise opacifiers and colorants selected from titanium dioxide, calcium carbonate, iron oxides, aluminium lakes or mixtures thereof. Suitable film coating can be present in an amount of 1% to 10% by weight relative to the total weight of the solid pharmaceutical composition. In a preferred embodiment, the film coating (iii) is in an amount of at least 1.5% up to 10% by weight relative to the total weight of the solid pharmaceutical composition. In a more preferred embodiment, the film coating (iii) is in an amount of at least 2% up to 10% by weight relative to the total weight of the solid pharmaceutical composition.

In a preferred embodiment, the film coating (iii) is prepared in an aqueous medium. The present invention, nevertheless, allows the presence of organic solvents such as short-chain alcohols, for example, methanol, chlorinated derivatives such as methylene chloride, acetone or mixtures thereof with water, as a medium for preparation of the film coating (iii), wherein the concentration of the organic solvent in the medium is no higher than 30%.

In a preferred embodiment, the pharmaceutical composition according to the first aspect of the present invention comprises a film coating (iii), which comprises a film forming agent and a filler as defined above. The presence of a filler in the film coating may improve the quality of the film and the ease of the handling during the manufacturing and packaging of said solid pharmaceutical composition, avoiding the peeling of the pharmaceutical composition. Said film coating is preferably prepared in an aqueous medium as defined above.

In a particular embodiment, the pharmaceutical composition according to the first aspect of the present invention comprises between 10% and 90% (w/w), preferably between 45% and 90% (w/w), more preferably between 60% and 90% (w/w) of the granulate (i); between 10% and 90% (w/w), preferably between 10% and 85% (w/w), more preferably between 10% and 35% (w/w) of the extragranular phase (ii); and optionally between 1% and 10% (w/w), preferably between 2% and 8% (w/w) of the film coating (iii). Weight percentage by weight relative to the weight of the pharmaceutical composition. The sum of all components adding to one hundred.

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process.

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant in an amount from 0.1% to 2.5% by weight relative to the total weight of the solid pharmaceutical composition; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process.

In a more preferred embodiment of the first aspect of the present invention the solid pharmaceutical composition comprises:
(i) a granulate comprising dexketoprofen trometamol, at least one lubricant; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process and the lubricant of the granulate (i) is the same as the lubricant of the extragranular phase (ii).

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process and the lubricant of the granulate (i) is the same as the lubricant of the extragranular phase (ii).

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant in an amount from 0.1% to 2.5% by weight relative to the total weight of the solid pharmaceutical composition; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process and the lubricant of the granulate (i) is the same as the lubricant of the extragranular phase (ii).

In a more preferred embodiment of the first aspect of the present invention the solid pharmaceutical composition comprises:
(i) a granulate comprising dexketoprofen trometamol and at least one lubricant; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process and the lubricant of the granulate (i) and/or the lubricant of the extragranular phase (ii) is magnesium stearate.

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process and the lubricant of the granulate (i) and/or the lubricant of the extragranular phase (ii) is magnesium stearate.

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant in an amount from 0.1% to 2.5% by weight relative to the total weight of the solid pharmaceutical composition; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process and the lubricant of the granulate (i) and/or the lubricant of the extragranular phase (ii) is magnesium stearate.

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant in an amount from 0.1% to 2.5% by weight relative to the total weight of the solid pharmaceutical composition; optionally at least one diluent and/or at least one binder and/or at least one disintegrant;
(ii) an extragranular phase comprising at least one diluent in an amount from 5% to 80% by weight relative to the total weight of the solid pharmaceutical composition, at least one disintegrant in an amount from 0.5% to 10% by weight relative to the total weight of the solid pharmaceutical composition and at least one lubricant in an amount from 0.1% to 2.5% by weight relative to the total weight of the solid pharmaceutical composition;
(iii) optionally a film coating in an amount from 1% to 10% by weight relative to the total weight of the solid pharmaceutical composition, which comprises a film forming agent;
wherein the granulate (i) is obtained by a dry granulation process.

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant in an amount from 0.1% to 2.5% by weight relative to the total weight of the solid pharmaceutical composition; optionally at least one diluent and/or at least one binder and/or at least one disintegrant;
(ii) an extragranular phase comprising at least one diluent in an amount from 5% to 80% by weight relative to the total weight of the solid pharmaceutical composition, at least one disintegrant in an amount from 0.5% to 10% by weight relative to the total weight of the solid pharmaceutical composition and at least one lubricant in an amount from 0.1% to 2.5% by weight relative to the total weight of the solid pharmaceutical composition;
(iii) optionally a film coating in an amount from 1% to 10% by weight relative to the total weight of the solid pharmaceutical composition, which comprises a film forming agent;
wherein the granulate (i) is obtained by a dry granulation process and the lubricant of the granulate (i) and/or the lubricant of the extragranular phase (ii) is magnesium stearate.

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant in an amount from 0.1% to 2.5% by weight relative to the total weight of the solid pharmaceutical composition; optionally at least one diluent and/or at least one binder and/or at least one disintegrant;
(ii) an extragranular phase comprising at least one diluent in an amount from 10% to 30% by weight relative to the total weight of the solid pharmaceutical composition, at least one disintegrant in an amount from 1% to 5% by weight relative to the total weight of the solid pharmaceutical composition and at least one lubricant in an amount from 0.2% to 2% by weight relative to the total weight of the solid pharmaceutical composition;
(iii) optionally a film coating in an amount from 1% to 10% by weight relative to the total weight of the solid pharmaceutical composition, which comprises a film forming agent;
wherein the granulate (i) is obtained by a dry granulation process.

In a more preferred embodiment of the first aspect of the present invention the solid pharmaceutical composition comprises:
(i) a granulate comprising dexketoprofen trometamol in an amount from 5% to 30% by weight relative to the total weight of the solid pharmaceutical composition, and at least one lubricant in an amount from 0.1% to 2.5% by weight relative to the total weight of the solid pharmaceutical composition; optionally at least one diluent and/or at least one binder and/or at least one disintegrant;
(ii) an extragranular phase comprising at least one diluent in an amount from 10% to 30% by weight relative to the total weight of the solid pharmaceutical composition, at least one disintegrant in an amount from 1% to 5% by weight relative to the total weight of the solid pharmaceutical composition and at least one lubricant in an amount from 0.2% to 2% by weight relative to the total weight of the solid pharmaceutical composition;
(iii) optionally a film coating in an amount from 1% to 10% by weight relative to the total weight of the solid pharmaceutical composition, which comprises a film forming agent;
wherein the granulate (i) is obtained by a dry granulation process and the lubricant of the granulate (i) and/or the lubricant of the extragranular phase (ii) is magnesium stearate.

In a more preferred embodiment of the first aspect of the present invention the solid pharmaceutical composition comprises:
(i) a granulate in an amount from 10% to 90% by weight relative to the total weight of the solid pharmaceutical composition;
(ii) an extragranular phase in an amount from 10% to 90% by weight relative to the total weight of the solid pharmaceutical composition;
(iii) optionally a film coating in an amount from 1% to 10% by weight relative to the total weight of the solid pharmaceutical composition;
wherein the granulate (i) is obtained by a dry granulation process.

In a more preferred embodiment of the first aspect of the present invention the solid pharmaceutical composition comprises:
(i) a granulate in an amount from 10% to 90% by weight relative to the total weight of the solid pharmaceutical composition;
(ii) an extragranular phase in an amount from 10% to 90% by weight relative to the total weight of the solid pharmaceutical composition;
(iii) optionally a film coating in an amount from 1% to 10% by weight relative to the total weight of the solid pharmaceutical composition;
wherein the granulate (i) is obtained by a dry granulation process and the lubricant of the granulate (i) and/or the lubricant of the extragranular phase (ii) is magnesium stearate.

The solid pharmaceutical composition of dexketoprofen trometamol of the present invention has a good dosage uniformity, which refers to pharmaceutical compositions with a good degree of uniformity with regards to the amount of the dexketoprofen trometamol among various dosage units. Preferably, the tablets of the present invention show a good acceptance value (AV) equal or lower than 10, more preferably equal or lower than 7, the most preferably equal or lower than 5. Additionally, the solid pharmaceutical composition of dexketoprofen trometamol of the present invention is characterised by having high polymorphic purity, together with low initial levels of degradation impurities and high chemical and polymorphic stability over time, even when it is packed in blisters made of standard materials which do not have a high resistance to moisture, such as polyvinyl chloride (PVC) or polyvinylidene chloride (PVdC). Thus, the present inventors have found that the solid pharmaceutical composition of the invention, initially containing equal or less than 0.3% (w/w), preferably equal or less than 0.1% (w/w), of total degradation impurities (measured by HPLC) (by weight of the total weight of the active pharmaceutical ingredient), were stable and kept the level of total degradation impurities equal or below 0.3% (w/w), preferably equal or below 0.1% (w/w) by weight of the total weight of the active pharmaceutical ingredient after at least 9 months at 30ºC and 65% Relative Humidity (RH). In addition, the polymorphic form remains stable. The stability of the solid pharmaceutical composition of dexketoprofen trometamol of the present invention was evaluated and calculated according to the pharmaceutical guideline ICH Q1A (R2) "Stability Testing of New Drug Substances and Products" of February 2003. The effect of temperature and storage conditions was tested. The stability tests were preferably performed initially (right after manufacturing) and after 3, 6, 9 and 12 months of storage of the pharmaceutical composition of the present invention. The pharmaceutical compositions of the present invention were packed in PVC/aluminium blisters. Tested storage conditions were 30°C ± 2°C temperature and 65 % ± 5% Relative Humidity (RH). In addition, it has been found that the solid pharmaceutical compositions according to the present invention, stored under said conditions of 30ºC/65% RH in PVC/aluminium blisters, contained low amounts of water initially and over time, preventing or delaying potential polymorphic transformation of dexketoprofen trometamol. The amount of water in the pharmaceutical composition of the present invention is measured by the loss on drying (LOD) values, as stated in the European Pharmacopeia 7.0. (2.2.32, monograph). Particularly, the LOD of the pharmaceutical composition of the present invention is measured in samples of approximately 1.5 g of crushed tablets, keeping the samples for 3 hours at 80°C in a vacuum drying oven, over diphosphorus pentoxide. Thus, accordingly to the present invention, the Loss on drying (LOD) values of the pharmaceutical compositions of the present invention were initially equal or below 8%, preferably equal or below 7%; said values being maintained after at least 9 months at 30ºC/65% RH. Thus, the solid pharmaceutical compositions of dexketoprofen trometamol of the present invention are very versatile and neither need to be packed in special and expensive containers nor to be stored in special packing conditions, such as in the presence of an inert atmosphere, in order to preserve the polymorphic and chemical purity of the dexketoprofen trometamol.

Another advantage of the solid pharmaceutical composition of dexketoprofen trometamol of the present invention is the very fast dissolution rate of the active ingredient dexketoprofen trometamol. Thus, the solid pharmaceutical composition of the present invention can exhibit a dissolution rate according to which at least 80% of the dexketoprofen trometamol is dissolved within 15 min or less and/or at least 70% of the dexketoprofen trometamol is dissolved within 10 min or less. In a preferred embodiment, at least 85% of the dexketoprofen trometamol is released within 15 min or less and/or at least 75% of the dexketoprofen trometamol is dissolved within 10 min or less. In a more preferred embodiment, at least 90% of the dexketoprofen trometamol is released within 15 min or less and/or at least 80% of the dexketoprofen trometamol is dissolved within 10 min or less. The dissolution rate was determined using an Apparatus 2 as stated in European Pharmacopeia 7.0.(2.9.3., monograph), placing the pharmaceutical composition according to the present invention within a vessel containing 900 mL of an hydrochloric acid solution pH= 1.2 at 37°C, and stirring the solution at 75 rpm with paddles.

A second aspect of the present invention is to provide a process for preparing the solid oral pharmaceutical composition of dexketoprofen trometamol of the invention, said process comprising the following steps:
1) Providing a granulate (i) which comprises the steps of:
   a) blending dexketoprofen trometamol and at least one lubricant;
   b) optionally, adding at least one diluent and/or at least one binder and/or at least one disintegrant;
   c) compressing the blended components of step (a) and (b), to obtain a compacted blend;
   d) reducing the size of the compacted blend obtained in step c) to obtain the granulate (i);
2) mixing the granulate obtained in step (1) with at least one diluent and/or at least one disintegrant;
3) mixing at least one lubricant with the blend obtained in step (2);
4) compressing the blend obtained in step (3) to form a tablet;
5) optionally, coating the tablet obtained in step (4) with a film coating comprising a film forming agent;
wherein the step (1) is carried out by a dry granulation process.

The components used in the process according to the second aspect of the present invention, as defined above, can be sieved prior to the blending/mixing steps.

The specific components used in the process for the preparation of the solid oral pharmaceutical composition of dexketoprofen trometamol of the present invention, as well as their properties and amounts, are as described above for the first aspect of the invention.

The dry granulation process of step (1), according to the second aspect of the present invention, may be performed by using conventional dry granulation procedures, such as slugging or roller compaction processes. Said dry granulation process allows obtaining solid oral pharmaceutical compositions according to the first aspect of the present invention, which contain low amounts of water initially and over time, preventing or delaying potential polymorphic transformation of dexketoprofen trometamol, as defined above for the first aspect of the present invention.

In a preferred embodiment of the second aspect of the present invention, the lubricant used in step (1a) is the same as the lubricant used in step (3).

According to another preferred embodiment of the second aspect of the present invention, the lubricant used in both step (1 a) and step (3) is magnesium stearate.

In another preferred embodiment of the second aspect of the present invention, the step (1d) is carried out by milling.

In a particular embodiment of the second aspect of the present invention, the granulate (i) obtained in step (1) may be also be mixed with a binder in step (2).

In a preferred embodiment of the second aspect of the present invention, the tablet obtained in step (4) is further coated with a film coating comprising a film forming agent. The film forming agent, toghether with other possible components of said film coating are as described above for the first aspect of the present invention. Additionally, the amount and the preparation of said film coating must also to be understood as described above for the fisrt aspect of the present invention. The coating process of step (5), according to the second aspect of the present invention, may be performed by using conventional coating procedures and equipment, such as spraying or spray-drying by means of coating pan, spray or fluidized coating equipment. Preferably said coating process is performed by spraying using a coating pan.

The present invention also provides a solid oral pharmaceutical composition of dexketoprofen trometamol, which is obtained or obtainable by the process of the present invention.

The solid oral pharmaceutical composition of dexketoprofen trometamol prepared according to the process of the invention preferably has the characteristics of the solid oral pharmaceutical composition of the present invention.

In addition, the present invention provides a solid oral pharmaceutical composition of dexketoprofen trometamol as defined above for use in therapy.

The present invention also provides a solid oral pharmaceutical composition of dexketoprofen trometamol as defined above for use in the treatment of pain and inflammation.

Also provided is a method of treatment of pain and inflammation, the method comprising the administration, to a subject in need of such treatment, of a solid oral pharmaceutical composition of dexketoprofen trometamol as defined above.

In another aspect, the present invention relates to the use of a solid oral pharmaceutical composition of dexketoprofen trometamol as defined above for the treatment of pain and inflammation.

In the following, the present invention is further illustrated by examples. They should in no case be interpreted as a limitation of the scope of the invention as defined in the claims. Unless indicated otherwise, all indications of percentage are by weight and temperatures are in degrees Celsius.

### DESCRIPTION OF PREFERRED EMBODIMENTS

The present invention in particular is directed to the following embodiments:
1. A solid oral pharmaceutical composition comprising:
   (i) a granulate comprising dexketoprofen trometamol and a at least one lubricant; optionally at least one diluent and/or at least one binder and/or at least one disintegrant;
   (ii) an extragranular phase comprising at least one diluent, at least one disintegrant and at least one lubricant;
   (iii) optionally a film coating comprising a film forming agent;
      wherein the granulate (i) is obtained by a dry granulation process.
2. A solid pharmaceutical composition according to the first embodiment, wherein the pharmaceutical composition is in the form of a tablet.
3. A solid pharmaceutical composition according to any of the preceding embodiments, wherein the dexketoprofen trometamol of the granulate (i) is in an amount from 5% to 30% by weight relative to the total weight of the solid pharmaceutical composition.
4. A solid pharmaceutical composition according to any of the preceding embodiments, wherein the lubricant of the granulate (i) is in an amount from 0.1% to 2.5% by weight relative to the total weight of the solid pharmaceutical composition.
5. A solid pharmaceutical composition according to any of the preceding embodiments, wherein the lubricant of the granulate (i) or the lubricant of the extragranular phase (ii) is selected from magnesium stearate, glyceryl palmitostearate, sucrose stearate and mixtures thereof.
6. A solid pharmaceutical composition according to any of the preceding embodiments, wherein the diluent of the granulate (i) or the diluent of the extragranular phase (ii) is selected from microcrystalline cellulose, silicified microcrystalline cellulose, maize starch, pregelatinized starch, and mixtures thereof.
7. A solid pharmaceutical composition according to any of the preceding embodiments, wherein the diluent of the extragranular phase (ii) is in an amount from 4% to 80% by weight relative to the total weight of the solid pharmaceutical composition.
8. A solid pharmaceutical composition according to any of the preceding embodiments, wherein the diluent of the granulate (i) is in an amount of 0% to 80% by weight relative to the total weight of the solid pharmaceutical composition.
9. A solid pharmaceutical composition according to any of the preceding embodiments, wherein the disintegrant of the granulate (i) or the disintegrant of the extragranular phase (ii) is selected from pregelatinized starch, sodium starch glycolate, croscarmellose or salts thereof, crospovidone, and mixtures thereof.
10. A solid pharmaceutical composition according to any of the preceding embodiments, wherein the disintegrant of the extragranular phase (ii) is in an amount from 0.5% to 10% by weight relative to the total weight of the solid pharmaceutical composition.
11. A solid pharmaceutical composition according to any of the preceding embodiments, wherein the disintegrant of the granulate (i) is in an amount from 0% to 20% by weight relative to the total weight of the solid pharmaceutical composition.
12. A solid pharmaceutical composition according to any of the preceding embodiments, wherein the lubricant of the extragranular phase (ii) is in an amount from 0.1% to 2.5% by weight relative to the total weight of the solid pharmaceutical composition.
13. A solid pharmaceutical composition according to any of the preceding embodiments, wherein the lubricant of the granulate (i) is the same as the lubricant of the extragranular phase (ii).
14. A solid pharmaceutical composition according to any of the preceeding embodiments, wherein the extragranular phase (ii) comprises a binder.
15. A solid pharmaceutical composition according to any of the preceding embodiments, wherein the binder of the granulate (i) or the binder of the extragranular phase (ii) is selected from starch, pregelatinized starch, povidone and mixtures thereof.
16. A solid pharmaceutical composition according to any of the preceding embodiments, wherein the binder of the granulate (i) or the binder of the extragranular phase (ii) is in an amount from 0% to 15% by weight relative to the total weight of the solid pharmaceutical composition.
17. A solid pharmaceutical composition according to any of the preceding embodiments, wherein the film coating (iii) comprises a film forming agent selected from cellulose derivatives methylcellulose, carboxymethylcellulose, sodium carboxymethylcellulose, hydroxymethylcellulose, hydroxypropylcellulose, hydroxyethyl cellulose, or hydroxypropyl methylcellulose, and mixtures thereof.
18. A solid pharmaceutical composition according to the preceding embodiment, wherein the film forming agent is hydroxypropyl methylcellulose.
19. A solid pharmaceutical composition according to any of the preceding embodiments, wherein the granulate (i) is in an amount between 10% and 90% by weight relative to the weight of the pharmaceutical composition; and the extragranular phase (ii) is in an amount between 10% and 90% by weight relative to the weight of the pharmaceutical composition.
20. A solid pharmaceutical composition according to the preceding embodiment, which also comprises a film coating (iii) in an amount between 1% and 10% by weight relative to the weight of the pharmaceutical composition.
21. A process for the preparation of a solid oral pharmaceutical composition of dexketoprofen trometamol, comprising the steps of:
   1) providing a granulate (i) which comprises the steps of:
      a) blending dexketoprofen trometamol and at least one lubricant;
      b) optionally, adding at least one diluent and/or at least one binder and/or at least one disintegrant;
      c) compressing the blended components of step (a) and (b), to obtain a compacted blend;
      d) reducing the size of the compacted blend obtained in step (c) to obtain the granulate (i);
   2) mixing the granulate obtained in step (1) with at least one diluent and/or at least one disintegrant;
   3) mixing at least one lubricant with the blend obtained in step (2);
   4) compressing the blend obtained in step (3) to form a tablet;
   5) optionally, coating the tablet obtained in step (4) with a film coating comprising a film forming agent;
   wherein the step (1) is carried out by a dry granulation process.
22. A process according to the preceding embodiment, wherein the granulate (i) obtained in step (1) may be also be mixed with at least one binder in step (2).
23. A process according to the two preceding embodiments, wherein the dexketoprofen trometamol of step (1 a) and the lubricant of step (1 a) and (3) are sieved prior to blending.
24. A process according to any of the embodiments 21 to 23, wherein the diluent, the binder and the disintegrant of step (1b), and the diluent, the binder and the disintegrant of step (2) are sieved prior to blending.
25. A process according to any of the embodiments 21 to 24, wherein the lubricant used in step (1a) and/or step (3) is selected from magnesium stearate, glyceryl palmitostearate, sucrose stearate and mixtures thereof.
26. A process according to any of the embodiments 21 to 25, wherein the diluent used in step (1b) and/or step (2) is selected from microcrystalline cellulose, silicified microcrystalline cellulose, maize starch, pregelatinized starch, and mixtures thereof.
27. A process according to any of the embodiments 21 to 26, wherein the binder used in step (1b) and/or step (2) is selected from is selected from starch, pregelatinized starch, povidone and mixtures thereof.
28. A process according to any of the embodiments 21 to 27, wherein the disintegrant used in step (1b) and/or step (2) is selected from pregelatinized starch, sodium starch glycolate, croscarmellose or salts thereof, crospovidone, and mixtures thereof.
29. A process according to any of the embodiments 21 to 28, wherein the lubricant of step (1 a) is the same as the the lubricant of step (3).
30. A process according to any of the embodiments 21 to 29, wherein the step (1 d) is carried out by milling.
31. A process according to any of the embodiments 21 to 30, wherein the film coating of step (4) comprises a film forming agent selected from cellulose derivatives methylcellulose, carboxymethylcellulose, sodium carboxymethylcellulose, hydroxymethylcellulose, hydroxypropylcellulose, hydroxyethyl cellulose, or hydroxypropyl methylcellulose, and mixtures thereof.
32. A solid pharmaceutical composition obtained or obtainable by the process of any of embodiments 21 to 31.
33. The solid pharmaceutical composition according to embodiments 1 to 20, for use in therapy.
34. The solid pharmaceutical composition according to embodiments 1 to 20, for use in the treatment of pain and inflammation.
35. The use of a solid pharmaceutical composition according to embodiments 1 to 20 for the treatment of pain and inflammation.
36. A method of treatment of pain and inflammation, the method comprising the administration, to a subject in need of such treatment, of a solid pharmaceutical composition according to claims 1 to 20.

### EXAMPLES

### Example 1: pharmaceutical composition prepared by direct compression according to EP 1739072A1

| **Ingredients** | **Content (mg/composition)** |
|---|---|
| Dexketoprofen trometamol (Form A) (d₉₀= 20.4µm, d₅₀= 6.8µm) | 36.90 |
| Maize starch | 6.70 |
| Microcrystalline cellulose | 10.00 |
| Lactose | 196.00 |
| Magnesium stearate | 10.40 |
| **Total composition (mg)** | **260.00** |

The inventors of the present invention tried to prepare dexketoprofen trometamol form A tablets by direct compression as described in the application patent EP 1739072 A1. Thus, they mixed dexketoprofen trometamol, lactose, maize starch and microcrystalline cellulose; followed by the addition of magnesium stearate as disclosed in said application patent. They tried to subject the mixture obtained by such a process to direct compression, but they found that said mixture is unsuitable for tableting by direct compression since the flowability of said mixture is severely impaired (it does not flow even when the funnel has a diameter of 25 mm), which made said formulations unsuited for industrial applications. Therefore, tablets prepared by direct compression cannot be obtained.

### Example 2: pharmaceutical composition prepared by direct compression (comparative example)

| **Ingredients** | **Content (mg/composition)** |
|---|---|
| Dexketoprofen trometamol (Form A) (d₉₀= 103.64µm, d₅₀= 12.66µm) | 36.90 |
| Pregelatinized starch | 52.53 |
| Microcrystalline cellulose | 157.58 |
| Sodium starch glycolate | 10.40 |
| Magnesium stearate | 2.60 |
| **Total composition (mg)** | **260.00** |

Dexketoprofen trometamol was mixed with pregelatinized starch, microcrystalline cellulose and sodium starch glycolate; followed by the addition of magnesium stearate. The mixture obtained showed very poor flow properties that made it unsuitable for tableting by direct compression (it does not flow even when the funnel has a diameter of 25 mm). Therefore, tablets prepared by direct compression cannot be obtained.

### Example 3:

### Formulation composition

| **Ingredients:** | **Content:** |
|---|---|
| **Granulate** | **mg/Tablet** |
| Dexketoprofen trometamol (d₉₀= 60.34 µm; d₅₀= 9.08 µm) | 36.90 |
| Microcrystalline cellulose | 127.40 |
| Pregelatinised starch | 26.26 |
| Sodium starch glycolate (type A) | 5.20 |
| Magnesium stearate | 2.60 |

| **Extragranular phase** | |
|---|---|
| Microcrystalline cellulose | 55.14 |
| Sodium starch glycolate (type A) | 5.20 |
| Magnesium stearate | 1.30 |
| **Total tablet** | **268.00** |

### Manufacturing process

1. Dexketoprofen trometamol, pregelatinised starch, magnesium stearate, part of the microcrystalline cellulose (127.40 mg) and part of the sodium starch glycolate (type A) (5.20 mg) were sieved and mixed.
2. The powder blend obtained in step 1 was dry granulated by slugging with a tableting machine, followed by milling of the slugs to provide a granulate.
3. The remaining microcrystalline cellulose (55.14 mg) and sodium starch glycolate (type A) (5.20 mg) were sieved and blended with the granulate obtained in step 2.
4. The remaining magnesium stearate (1.30 mg) was sieved and blended with the blended obtained in step 3.
5. The blend obtained in step 4 was compressed to provide a tablet.

The film-coated tablets obtained in example 3 were tested for dissolution and compared with the commercially available product Enantyum@ (25 mg dexketoprofen trometamol). The data are shown below.

Dissolution conditions: 900 mL of hydrochloric acid media pH 1.2, Apparatus 2 (Pharmacopeia Europea 7.0., 2.9.3.), 75 rpm stirring

| **Time (minutes)** | **Cumulative % of dexketoprofen trometamol dissolved** | |
|---|---|---|
| | **Example 3** | **Enantyum** |
| 10 | 84 | 73 |
| 15 | 92 | 89 |
| 20 | 95 | 97 |
| 30 | 97 | 97 |

### Example 4:

### Formulation composition

| **Ingredients:** | **Content:** |
|---|---|
| **Granulate** | **mg/Tablet** |
| Dexketoprofen trometamol (d₉₀= 122.17 µm; d₅₀= 10.69 µm) | 36.90 |
| Microcrystalline cellulose | 157.58 |
| Pregelatinised starch | 52.53 |
| Sodium starch glycolate (type A) | 5.20 |
| Magnesium stearate | 1.30 |

| **Extragranular phase** | |
|---|---|
| Sodium starch glycolate (type A) | 5.20 |
| Magnesium stearate | 1.30 |

| **Coating** | |
|---|---|
| Opadry Y-1-7000 White* | 7.80 |
| **Total tablet** | **267.80** |

| | |
|---|---|
| ** Opadry Y-1-7000 White is a commercially available pre-formulated coating material based on hydroxypropyl methylcellulose (HPMC)* | |

### Manufacturing process

1. Dexketoprofen trometamol, pregelatinised starch, magnesium stearate, microcrystalline cellulose and part of the sodium starch glycolate (type A) (5.20 mg) were sieved and mixed.
2. The powder blend obtained in step 1 was dry granulated by slugging with a tableting machine, followed by milling of the slugs to provide a granulate.
3. The remaining sodium starch glycolate (type A) (5.20 mg) were sieved and blended with the granulate obtained in step 2.
4. The remaining magnesium stearate (1.30 mg) was sieved and blended with the blended obtained in step 3.
5. The blend obtained in step 4, having a flowability of 18 seconds (funnel 15 mm), was compressed to provide a tablet.
6. The tablet obtained in step 5 was coated with a 15% w/w aqueous dispersion of Opadry Y-1-7000, by spraying the dispersion over the tablet using a coating pan system

The film-coated tablets of example 4, packed in polyvinyl chloride (PVC)/ aluminum blisters and exposed to stress stability testing at 30°C and 65% relative humidity (RH), showed to be stable after at least 9 months, keeping the level of total degradation impurities equal to or below 0.1 % (w/w) by weight of the total weight of the dexketoprofen trometamol.

### Example 5:

### Formulation composition

| **Ingredients:** | **Content:** |
|---|---|
| **Granulate** | **mg/Tablet** |
| Dexketoprofen trometamol (d₉₀= 60.34 µm; d₅₀= 9.08 µm) | 36.90 |
| Microcrystalline cellulose | 127.40 |
| Pregelatinised starch | 26.26 |
| Sodium starch glycolate (type A) | 5.20 |
| Magnesium stearate | 2.60 |

| **Extragranular phase** | |
|---|---|
| Microcrystalline cellulose | 55.14 |
| Sodium starch glycolate (type A) | 5.20 |
| Magnesium stearate | 1.30 |

| **Coating** | |
|---|---|
| Opadry OY-GM-7305 White* | 8.00 |
| **Total tablet** | **268.00** |

| | |
|---|---|
| ** Opadry OY-GM-7350 White is a commercially available pre-formulated coating material based on hydroxypropyl methylcellulose (HPMC) and polydextrose* | |

### Manufacturing process

1. Dexketoprofen trometamol, pregelatinised starch, magnesium stearate, part of the microcrystalline cellulose (127.40 mg) and part of the sodium starch glycolate (type A) (5.20 mg) were sieved and mixed.
2. The powder blend obtained in step 1 was dry granulated by slugging with a tableting machine, followed by milling of the slugs to provide a granulate.
3. The remaining microcrystalline cellulose (55.14 mg) and sodium starch glycolate (type A) (5.20 mg) were sieved and blended with the granulate obtained in step 2.
4. The remaining magnesium stearate (1.30 mg) was sieved and blended with the blended obtained in step 3.
5. The blend obtained in step 4, having a flowability of 16.9 seconds (funnel 15 mm), was compressed to provide a tablet.
6. The tablet obtained in step 5 was coated with a 12% w/w aqueous dispersion of Opadry OY-GM-7350, by spraying the dispersion over the tablet using a coating pan system

The film-coated tablets obtained in example 5 are characterised by the following:
AV (acceptance value) = 3.4
LOD (Loss of Drying) (%) = 4.2%

The film-coated tablets obtained in example 5 were tested for dissolution and compared with the commercially available product Enantyum® (25 mg dexketoprofen trometamol). The data are shown below.

Dissolution conditions: 900 mL of hydrochloric acid media pH 1.2, Apparatus 2 (Pharmacopeia Europea 7.0., 2.9.3.), 75 rpm stirring

| **Time (minutes)** | **Cumulative % of dexketoprofen trometamol dissolved** | |
|---|---|---|
| | **Example 5** | **Enantyum** |
| 10 | 84 | 73 |
| 15 | 93 | 89 |
| 20 | 96 | 93 |
| 30 | 99 | 96 |

The film-coated tablets of example 5, packed in polyvinyl chloride (PVC)/ aluminum blisters and exposed to stress stability testing at 30°C and 65% relative humidity (RH), showed to be stable, keeping the level of total degradation impurities equal to 0.1 % (w/w) after 9 months, and equal to 0.2% (w/w) after 12 months. The LOD value after 12 months is equal to 6.2%.

### Example 6:

| **Ingredients:** | **Content:** |
|---|---|
| **Granulate** | **mg/Tablet** |
| Dexketoprofen trometamol (d₉₀= 20.06 µm; d₅₀= 6.79 µm) | 36.90 |
| Microcrystalline cellulose | 156.28 |
| Pregelatinised starch | 26.26 |
| Sodium starch glycolate (type A) | 5.20 |
| Magnesium stearate | 2.60 |

| **Extragranular phase** | |
|---|---|
| Microcrystalline cellulose | 26.26 |
| Sodium starch glycolate (type A) | 5.20 |
| Magnesium stearate | 1.30 |

| **Coating** | |
|---|---|
| Opadry OY-GM-7305 White* | 8.00 |
| **Total tablet** | **268.00** |

| | |
|---|---|
| ** Opadry OY-GM-7350 White is a commercially available pre-formulated coating material based on hydroxypropyl methylcellulose (HPMC) and polydextrose* | |

### Manufacturing process

The tablets of example 6 are prepared as previously described in example 5.

The film-coated tablets obtained in example 6 are characterised by the following:
AV (acceptance value) = 3.4
LOD (Loss of Drying) (%) = 4.8%

The coated tablets of example 6, packed in polyvinyl chloride (PVC)/ aluminum blisters and exposed to stress stability testing at 30°C and 65% relative humidity (RH), showed to be stable, keeping the level of total degradation impurities equal to 0.1% (w/w) after at least 3 months. The LOD value after 3 months is equal to 6.4%.

## Claims

1. A solid oral pharmaceutical composition comprising:
(i) a granulate comprising dexketoprofen trometamol and a at least one lubricant; optionally at least one diluent and/or at least one binder and/or at least one disintegrant;
(ii) an extragranular phase comprising at least one diluent, at least one disintegrant and at least one lubricant;
(iii) optionally a film coating comprising a film forming agent;
wherein the granulate (i) is obtained by a dry granulation process.

2. The solid pharmaceutical composition according to claim 1, wherein the pharmaceutical composition is in the form of a tablet.

3. The solid pharmaceutical composition according to claims 1 or 2, wherein the dexketoprofen trometamol of the granulate (i) is in an amount from 5% to 30% by weight relative to the total weight of the solid pharmaceutical composition, and/or wherein the lubricant of the granulate (i) is in an amount from 0.1% to 2.5% by weight relative to the total weight of the solid pharmaceutical composition.

4. The solid pharmaceutical composition according to any of the preceding claims, wherein the lubricant of the granulate (i) or the lubricant of the extragranular phase (ii) is selected from magnesium stearate, glyceryl palmitostearate, sucrose stearate and mixtures thereof, and/or wherein the diluent of the granulate (i) or the diluent of the extragranular phase (ii) is selected from microcrystalline cellulose, silicified microcrystalline cellulose, maize starch, pregelatinized starch, and mixtures thereof.

5. The solid pharmaceutical composition according to any of the preceding claims, wherein the diluent of the extragranular phase (ii) is in an amount from 4% to 80% by weight relative to the total weight of the solid pharmaceutical composition and/or wherein the diluent of the granulate (i) is in an amount of 0% to 80% by weight relative to the total weight of the solid pharmaceutical composition.

6. The solid pharmaceutical composition according to any of the preceding claims, wherein the disintegrant of the granulate (i) or the disintegrant of the extragranular phase (ii) is selected from pregelatinized starch, sodium starch glycolate, croscarmellose or salts thereof, crospovidone, and mixtures thereof.

7. The solid pharmaceutical composition according to any of the preceding claims, wherein the disintegrant of the extragranular phase (ii) is in an amount from 0.5% to 10% by weight relative to the total weight of the solid pharmaceutical composition, and/or wherein the disintegrant of the granulate (i) is in an amount from 0% to 20% by weight relative to the total weight of the solid pharmaceutical composition, and/or wherein the lubricant of the extragranular phase (ii) is in an amount from 0.1% to 2.5% by weight relative to the total weight of the solid pharmaceutical composition.

8. The solid pharmaceutical composition according to any of the preceding claims, wherein the lubricant of the granulate (i) is the same as the lubricant of the extragranular phase (ii).

9. The solid pharmaceutical composition according to any of the preceding claims, wherein the extragranular phase (ii) comprises a binder, and/or wherein the binder of the granulate (i) or the binder of the extragranular phase (ii) is selected from starch, pregelatinized starch, povidone and mixtures thereof.

10. The solid pharmaceutical composition according to any of the preceding claims, wherein the film coating (iii) comprises a film forming agent selected from cellulose derivatives methylcellulose, carboxymethylcellulose, sodium carboxymethylcellulose, hydroxymethylcellulose, hydroxypropylcellulose, hydroxyethyl cellulose, or hydroxypropyl methylcellulose, and mixtures thereof, and/or wherein a film coating (iii) is comprised in an amount between 1% and 10% by weight relative to the weight of the pharmaceutical composition.

11. The solid pharmaceutical composition according to any of the preceding claims, wherein the granulate (i) is in an amount between 10% and 90% by weight relative to the weight of the pharmaceutical composition; and the extragranular phase (ii) is in an amount between 10% and 90% by weight relative to the weight of the pharmaceutical composition.

12. A process for the preparation of a solid oral pharmaceutical composition of dexketoprofen trometamol, comprising the steps of:
1) providing a granulate (i) which comprises the steps of:
a) blending dexketoprofen trometamol and at least one lubricant;
b) optionally, adding at least one diluent and/or at least one binder and/or at least one disintegrant;
c) compressing the blended components of step (a) and (b), to obtain a compacted blend;
d) reducing the size of the compacted blend obtained in step (c) to obtain the granulate (i);
2) mixing the granulate obtained in step (1) with at least one diluent and/or at least one disintegrant;
3) mixing at least one lubricant with the blend obtained in step (2);
4) compressing the blend obtained in step (3) to form a tablet;
5) optionally, coating the tablet obtained in step (4) with a film coating comprising a film forming agent;
wherein the step (1) is carried out by a dry granulation process.

13. The process according to the preceding claim, wherein the dexketoprofen trometamol of step (1 a) and the lubricant of step (1 a) and (3) are sieved prior to blending, and/or wherein the diluent, the binder and the disintegrant of step (1b), and the diluent and the disintegrant of step (2) are sieved prior to blending.

14. A solid pharmaceutical composition obtained or obtainable by the process of any of claims 12 or 13.

15. The solid pharmaceutical composition according to claims 1 to 11 or 14, for use in the treatment of pain and inflammation.
